(19) Europäisches Patentamt / European Patent Office / Office européen des brevets

(11) **EP 3 121 263 A1**

(12) **EUROPEAN PATENT APPLICATION**
published in accordance with Art. 153(4) EPC

(43) Date of publication:
25.01.2017 Bulletin 2017/04

(21) Application number: 15764190.3

(22) Date of filing: 13.03.2015

(51) Int Cl.:
*C12M 1/34* (2006.01)  *C12Q 1/06* (2006.01)
*G01N 21/76* (2006.01)  *G01N 21/77* (2006.01)
*G01N 33/50* (2006.01)

(86) International application number:
PCT/JP2015/057544

(87) International publication number:
WO 2015/141594 (24.09.2015 Gazette 2015/38)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA

(30) Priority: 20.03.2014 JP 2014057498

(71) Applicant: Hitachi Plant Services Co., Ltd.
Tokyo 170-6034 (JP)

(72) Inventors:
• MIYASHITA Noe
Tokyo 100-8280 (JP)
• TANAKA Makoto
Tokyo 100-8280 (JP)

(74) Representative: Beetz & Partner mbB
Patentanwälte
Steinsdorfstraße 10
80538 München (DE)

(54) **MICROBE MEASUREMENT SYSTEM AND MICROBE MEASUREMENT METHOD**

(57) The present invention makes a microorganism measuring system set control reference values with higher accuracy than ever. Based on results of repeated CFU countings and results of repeated ATP measurements, a microorganism measuring system (1) of the present invention obtains a probability density function of ATP measured values in a normal condition; determines an alert reference value at which a desired probability of false positives is equal to or less than a probability, and an action reference value at which a desired probability of false negatives is equal to or less than a probability; and thereby controls the ATP measured values. For this reason, the microorganism measuring system (1) can set the control reference values with higher accuracy than ever, without being influenced by an error in the conversion of ATP contents into CFU counts. Accordingly, the microorganism measuring system (1) can achieve the microbiological control with desired control accuracy.

FIG. 1

EP 3 121 263 A1

## Description

### TECHNICAL FIELD

The present invention relates to a microorganism measuring system for measuring microorganisms in products and materials of medicines, foods and the like, as well as in manufacturing environments. The present invention relates particularly to a technique of setting control reference values and making management for controlling microorganisms by use of a rapid microbiological method such as an ATP method.

### BACKGROUND ART

In facilities required to be aseptic and biologically clean, such as various clinical medical facilities, food factories, pharmaceutical manufacturing factories and basic-research laboratories, the numbers of microorganisms in their products, materials and enviromental air are counted for the purpose of avoiding microorganisms contamination on the products, and infections to humans. A culture method is known as a method of counting the number of microorganisms. However, it is well known that the counting of the number of microorganisms using the culture method requires a vast amount of time and labor.

As the rapid microbiological method is faster and more sensitive than the culture method, methods have been proposed in which: components of microorganisms are uniquely marked with a luminescent reagent or a fluorescent reagent; and the number of microorganisms is obtained by measuring their luminescence intensities. Among them, the ATP method is a method using a chemical substance ATP (Adenosine Triphosphate) included in living cells as an indicator for counting the number of microorganisms. The ATP method can complete a measurement in a couple of minutes to tens of minutes.

The ATP method uses luciferin-luciferase luminous reaction. The ATP method includes: mixing a luminescence reagent including the substrate luciferin and the enzyme luciferase, and an ATP-containing sample solution extracted from cells of microorganisms to cause reaction; and obtaining an ATP content from an amount of luminescence produced by the reaction. Patent Literature 1 discloses a kit for measuring an amount of living bacteria using such luminous reaction.

Meanwhile, an amount of bacteria obtained using the culture method is expressed in CFU (Colony Forming Unit) which is the number of colonies formed on an agar medium. CFU counts tolerated in measurement objects and environments are specified by Japanese Pharmacopoeia, guidelines and the like. To offering highly-reliable products while complying with the specified CFU counts, the manufactures set and manage control reference values, based on their rule of thumb, at an alert-level for making a caution and an action-level for determining an anomaly and taking an action such as a re-test or suspension of a production line.

A method of counting the number of living bacteria using the kit disclosed in Patent Literature 1 is certainly effective in reducing a length of time needed for the measurement. However, actual microbiological control using results from the ATP method requires the control reference values in terms of the ATP content to be set appropriately.

An intracellular ATP content in a microorganism differs depending on the strain of the microorganism. In a case where the strain of the microorganism in a sample is known, the ATP content can be converted into a CFU using an analytical curve prepared using samples including known amounts of microorganisms.

Even in a case where multiple types of microorganisms are present in a sample, the ATP content can be determined using the ATP method after separating target microorganisms from the other microorganisms using an antibody specific to the target microorganisms, as disclosed in Patent Literature 2. However, this method cannot detect the other microorganisms even if they are included in the sample.

Meanwhile, Patent Literature 3 discloses a microbiological control method including: testing whether or not a deviation occurs based on CFU of model microorganisms to which an ATP measured value is converted using an arbitrary conversion coefficient set in advance; and concurrently testing whether or not the ATP measured value has increased suddenly, and whether or not any of a sudden increase and continuous increase occurs by comparing the measurement result with past measurement results.

### CITATION LIST

**Patent Literature**

Patent Literature 1: Japanese Patent No. 3773151
Patent Literature 2: Japanese Patent Application Publication No. 2006-81506
Patent Literature 3: Japanese Patent No. 5030015

## SUMMARY OF INVENTION

### Technical Problem

However, the methods disclosed in Patent Literatures 1 to 3 have difficulties in setting the control reference values appropriately for the following three reasons.

First, the CFU defines the amount of microorganisms by using the proliferative activity of the microorganisms as its indicator, while the ATP method uses a metabolism activity of the microorganisms as its indicator. Using physiological activities as the indicators, the CFU and the ATP content have somewhat correlation therebetween, but the conversion of the ATP content into the CFU causes error because the activities used as their indicators are different in type.

Second, although the intracellular ATP content in a biological cell differs depending on the strain of the microorganism, there is the case that the intracellular ATP content even in the same strain of microorganism differs largely depending on the active state. For this reason, there may be a case where a change in the amount of microorganisms cannot be determined appropriately, such for example as a case where even if microorganisms whose ATP content is extremely less than a set conversion coefficient proliferate, the proliferation cannot be detected.

Third, generally speaking, the natural ecological system always contains multiple types of microorganisms, and one may consider that the same applies to products, materials and environmental air as measurement objects. However, the types of the microorganisms contained and their proportions differ from one environment to another, and do not remain unchanged even in the same environment. For this reason, even if a conversion coefficient for a general microorganism species is used, the conversion of the ATP measured value into CFU using such fixed conversion coefficient is highly likely to cause large error.

Because of many error factors and uncertainties as mentioned above, there have been problems that: it is difficult to set the control reference values for microorganisms appropriately; and it is impossible to know what range the control accuracy falls within.

An object of the present invention is to: set control reference values more accurately than ever in a system for measuring microorganisms in products, materials and their manufacturing environments using a rapid microbiological method, such as the ATP method, which uses measurement units different from those used in the conventional culture method; make the control accuracy fall within a desired range; and achieve microbiological control using the same.

### Solution to Problem

To solve the above problems, a microorganism measuring system according to claim 1 includes luminous reaction means for making ATP in cells of microorganisms in a sample cause luminous reaction by supplying a predetermined reagent to the microorganisms; optical measurement means for measuring luminescence intensities of the microorganisms causing the luminous reaction; arithmetic control means for converting measurement values of the luminescence intensities measured using the optical measurement means into ATP contents, storing the conversion results, and performing a statistical process. The arithmetic control means executes the steps of: inputting results of CFU countings repeatedly performed using a culture method in a normal condition; inputting results of ATP measurements repeatedly performed using the luminous reaction means and the optical measurement means at the same time as the CFU countings are repeatedly performed; based on the results of the repeated CFU countings, determining a coefficient of a probability density function of CFU counts in the sample; based on the results of the repeated ATP measurements, determining coefficients of a probability density function of ATP contents per CFU; using the determined coefficients of the two probability density functions, determining a probability density function of ATP measured values in the normal condition; performing a statistical test on appropriateness of the determined probability density function; determining a probability density function of ATP measured values in a contaminated condition corresponding to CFU counts in the contaminated condition set in advance; using the determined probability density function of the ATP measured values in the normal condition, calculating a alert reference value at which a probability of false positives is equal to or less than a probability set in advance; using the determined probability density function of the ATP measured values in the contaminated condition, calculating a warning reference value at which a probability of false negatives is equal to or less than a probability set in advance; and each time the ATP measurement is performed, testing whether or not the ATP measured value satisfies any of the alert reference value and the warning reference value.

In addition, a microorganism measuring system according to claim 2 includes the microorganism measuring system according to claim 1, in which a plurality of types of the probability density function of the ATP measured values are prepared, and the arithmetic control means further executes the step of, based on the results of the repeated CFU countings, selecting one from the plurality of types of the probability density function of the ATP measured values.

In addition, a microorganism measuring system according to claim 3 includes the microorganism measuring system according to claim 1 or 2, in which the arithmetic control means determines the probability density function of the ATP measured values in the contaminated condition using the same coefficients as those in the probability density function of the ATP measured values in the normal condition.

In addition, a microorganism measuring system according to claim 4 includes the microorganism measuring system according to claim 1 or 2, in which the arithmetic control means determines the probability density function of the ATP measured values in the contaminated condition using a mean ATP content and a standard deviation, obtained in advance using standard strain of bacteria, as the coefficients.

In addition, a microorganism measuring system according to claim 5 includes the microorganism measuring system according to claim 1, in which the arithmetic control means further executes the step of testing whether or not a volume of the stored results of the repeated CFU countings and a volume of the stored results of the repeated ATP measurements are enough for the statistical test.

In addition, a microorganism measuring system according to claim 6 includes the microorganism measuring system according to claim 1, in which the arithmetic control means further uses a probability density function of ATP measured values calculated using the results of the measurements in a predetermined period, as the probability density function of the ATP measured values.

In addition, a microorganism measuring system according to claim 7 includes the microorganism measuring system according to claim 1, further including a data input unit for inputting data and control conditions by manipulating keys. The arithmetic control means further uses the CFU counts in the contaminated condition, the probability of false positives, and the probability of false negatives, which are inputted using the data input unit.

In addition, a microorganism measuring system according to claim 8 includes the microorganism measuring system according to claim 1, further including a data input unit for inputting data and control conditions by manipulating keys. When performing the ATP measurement, the arithmetic control means further uses information on the sample inputted using the data input unit.

In addition, a microorganism measuring system according to claim 9 includes the microorganism measuring system according to claim 1, in which the arithmetic control means further executes the step of performing an alert procedure or an action procedure based on a result of the step of testing whether or not the ATP measured value satisfies any of the alert reference value and the action reference value each time the ATP measurement is performed.

In addition, a microorganism measuring system according to claim 10 includes the microorganism measuring system according to claim 1, further including printing means for outputting the results of the measurements in print. The arithmetic control means further executes the step of outputting the results of the ATP measurements and information needed to analyze the results of the ATP measurements using the printing means.

In addition, a microorganism measuring system according to claim 11 includes the microorganism measuring system according to claim 1, in which the arithmetic control means further executes the step of creating information needed to analyze the results of the ATP measurements, both in a protected format which disables the information from being tampered and in an unprotected format which enables the information to be analyzed.

## Advantageous Effects of Invention

According to the present invention, the microorganism measuring system for measuring microorganisms in products, materials, their production environments using a rapid microbiological method, such as an ATP method, whose measurement units are different from those of a conventional culture method is capable of: setting control reference values with higher accuracy than ever, and making their control accuracy fall within a desired range; and accordingly achieving microbiological control using them. Its details are as follows.

According to aspects of the invention as recited in claim 1 and 12, based on the results of the repeated CFU countings and the results of the repeated ATP measurements, the probability density function of the ATP measured values in the normal condition is obtained; the alert reference value at which the desired probability of false positives is equal to or less than the probability, and the action reference value at which the desired probability of false negatives is equal to or less than the probability are determined; and the ATP measured values are thereby controlled. For this reason, the control reference values can be set with higher accuracy than ever, without being influenced by an error in the conversion of the ATP contents into the CFU counts. Accordingly, the microbiological control can be fulfilled with desired control accuracy.

In addition, according to the aspects of the invention as recited in claim 1 and 12, the operation is carried out using the obtained probability density functions whose appropriateness is statistically tested. For this reason, differences between the probability distribution models (probability density functions) and actual probability distributions can fall within a predetermined range. Accordingly, the microorganisms can be controlled with the control reference values set with higher accuracy than ever.

One may consider that when an amount of microorganisms in a measurement object is small, types of microorganisms in the measurement object, their proportions and their activity states exert relatively large influences. Accordingly, ATP measured values obey a probability distribution which is different from that for a case where an amount of microorganisms is sufficiently large.

Accordingly to an aspect of the invention as recited in claim 2, one is selected from the types of probability distribution models (probability density functions) of the ATP measured values, based on the results of the repeated CFU countings. For this reason, an optimal probability distribution model of the amounts of microorganisms can be set based on the mean CFU count in the measurement object. Accordingly, the microorganisms can be controlled with the control reference values set with higher accuracy than ever.

When the measurement object is a closed environment such as a clean room, or an environment whose characteristic property such as pH has an effect on the growth of microorganisms, types of microorganisms detected from such an environment may be limited to a certain extent. In this case, it is expected that the numbers of bacteria increase while the types of bacteria and their proportions are not different between the normal condition and the contaminated condition of the measurement object.

According to an aspect of the invention as recited in claim 3, the probability density function of the ATP measured values in the contaminated condition is determined using the same coefficients as are used in the probability density function of the ATP measured values in the normal condition. For this reason, the microorganisms can be controlled with the control reference values set with higher accuracy than ever for the ATP measurement of the measurement object in which states of microorganisms are not different between the normal condition and the contaminated condition.

Meanwhile, there is high likelihood that the types of microorganisms in the measurement object and their proportions are largely different between the normal condition and the contaminated condition, since like in the general ecological system, the types of microorganisms and their proportions are different from one environment to another, and do not remain unchanged even in the same environment, as described above.

According to an aspect of the invention as recited in claim 4, the probability density function of the ATP measured values in the contaminated condition is determined using the mean ATP content and the standard deviation, obtained in advance using the standard strain of bacteria, as the coefficients. For this reason, the microorganisms can be controlled with the control reference values set with higher accuracy than ever for the ATP measurement of the measurement object in which the states of microorganisms are not different between the normal condition and the contaminated condition.

In the microorganism measuring system of the present invention, in the process of repeating the CFU countings and the ATP measurements, the probability distribution models (probability density functions) of the ATP measured values are constructed based on the detected data. For this reason, even though obtained from a cleaner condition where the number of detected bacteria is smaller, the probability density functions become largely different from the actual probability distributions.

According to an aspect of the invention as recited in claim 5, when the repeated ATP measurements are completed, it is tested whether or not the volume of the accumulated data are large enough for the statistical test for checking the appropriateness of the probability density functions obtained using the accumulated data. For this reason, the probability distribution models are unlikely to be constructed using a small volume of data, and to have large error. Accordingly, the microorganisms can be controlled with the control reference values set with higher accuracy than ever.

There is likelihood that the probability distribution of the ATP measured values in the measurement object changes, for example, after the facility returns from a break (maintenance), or due to factors such as seasonal changes, since like in the general ecological system, the types of microorganisms in the measurement object and their proportions are different from one environment to another, and do not remain unchanged even in the same environment, as described above.

According to an aspect of the invention as recited in claim 6, the arithmetic control means uses the probability density function of ATP measured values calculated using the results of the measurements in the predetermined period. For this reason, the current and past functions can be compared. Accordingly, the microorganisms can be controlled with higher accuracy than ever by: reviewing the long-term changes in the ATP measured values as described above; and resetting the control reference values depending on the necessity.

Generally speaking, the control reference values are set according to how large a risk of product contamination and a risk of human infection are. The control accuracy required for each control reference value also differs depending on how large the risks are.

According to an aspect of the invention as recited in claim 7, the arithmetic control means uses the CFU counts in the contaminated condition, the probability of false positives, and the probability of false negatives, which are inputted using the data input unit. For this reason, the control reference values can be set for each measurement object, and the microbiological control operation can be performed individually on the measurement object. Accordingly,

As is often the case with a facility required to be aseptic or biologically clean, microorganisms are measured in a variety of things such as products, materials and environments. It is not rare that multiple sampling positions are set in the same measurement object. If results of the measurements and information on the measured objects are recorded by being associated with each other inappropriately, there is likelihood that the results of the measurements are examined in an inappropriate manner.

In a case where the microorganism measuring method using reagents, such as the ATP method, is used, information on lots of the reagents used for the measurement, and the like need to be traceable for the purpose of appropriate quality control of products.

According to an aspect of the invention as recited in claim 8, when performing the ATP measurement, the arithmetic control means uses the information on the sample, such as information on the type of the measurement object, information on the sampling positions and information on the lots of the reagents, inputted using the data input unit. For this reason, there is no risk of recording the results of the measurements and the information on the sample by associating the results of measurements and the information on the sample with each other inappropriately. Accordingly, the microorganisms can be controlled with higher accuracy than ever.

In the microbiological control in the facility required to be aseptic or biologically clean, if biological contamination occurs, prompt action is required to be taken for the purpose of minimizing the damage.

According to an aspect of the invention as recited in claim 9, the alert procedure or the action procedure is activated based on whether or not the ATP measured value satisfies the control reference values. For this reason, in compliance with control standards set in advance, a prompt action can be taken without human judgement if the ATP measured value deviates from the control reference values. Accordingly, the microorganisms can be controlled with higher accuracy than ever.

Generally speaking, the results of the measurements in the microbiological control are important to a manufacturing facility which is required to apply high quality assurance to its products such as medicines. If the results of the measurements are tampered, lost, etc., appropriate quality assurance can be achieved.

According to an aspect of the invention as recited in claim 10, the results of the ATP measurements and the information needed to analyze the results of the ATP measurements are outputted by being printed on sheets. For this reason, there is no room for tampering. Accordingly, the microorganisms can be controlled with higher accuracy than ever.

In addition, according to an aspect of the invention as recited in claim 11, the results of the ATP measurements and the information needed to analyze the results of the ATP measurements are saved as a data file in the protected format. For this reason, there is no risk that the results and the information are tampered or lost. Accordingly, the microorganisms can be controlled with higher accuracy than ever.

Meanwhile, in the facility required to be aseptic or biologically clean, the microbiological control may be analyzed from more diversified viewpoints using the data on the microorganism measurements for the purpose of enhancing the quality of the microbiological control by improving the microbiological control constantly.

According to an aspect of the invention as recited in claim 11, the results of the ATP measurements and the information needed to analyze the results of the ATP measurements can be saved as a data file in the unprotected format, in addition to as the data file for data-recording and safekeeping purposes. For this reason, analyses and examinations can be freely performed using the data file in the unprotected format. Accordingly, the microorganisms can be controlled with higher accuracy than ever.

## BRIEF DESCRIPTION OF DRAWINGS

FIG.1 is an explanatory diagram of a configuration of a microorganism measuring system of an embodiment of the present invention.

FIG. 2 is an explanatory diagram showing a relationship among false positive, false negative and control reference values in the microorganism measuring system of the embodiment of the present invention.

FIG. 3A is a first half of a flowchart showing an outline of how to set the control reference values in the microorganism measuring system of the embodiment of the present invention.

FIG. 3B is a second half of the flowchart showing the outline of how to set the control reference values in the microorganism measuring system of the embodiment of the present invention.

FIG. 4 is a flowchart showing an outline of how to operate the microorganism measuring system of the embodiment of the present invention.

FIG. 5A is a schematic explanatory diagram of a long-term change analysis graph (Format A) of the microorganism measuring system of the embodiment of the present invention.

FIG. 5B is a schematic explanatory diagram of the long-term change analysis graph (Format B) of the microorganism measuring system of the embodiment of the present invention.

FIG. 6 is a schematic diagram for explaining a report on a result in print of the microorganism measuring system of the embodiment of the present invention.

FIG. 7 is a schematic diagram showing how data are stored in the microorganism measuring system 1 of the embodiment of the present invention, and how the output result files are configured.

**DESCRIPTION OF EMBODIMENTS**

Hereinafter, embodiments of the present invention will be described with reference to the accompanying drawings as needed.

The descriptions start with an overall configuration of a microorganism measuring system of the embodiment of the present invention, which will be followed by how a microorganism measuring system works. Thereafter, a probability distribution model of ATP measured values will be described. Finally, the descriptions will be provided for a procedure for setting actual control reference values using the probability distribution model, and how to use the thus-set control reference values.

<Microorganism Measuring System>

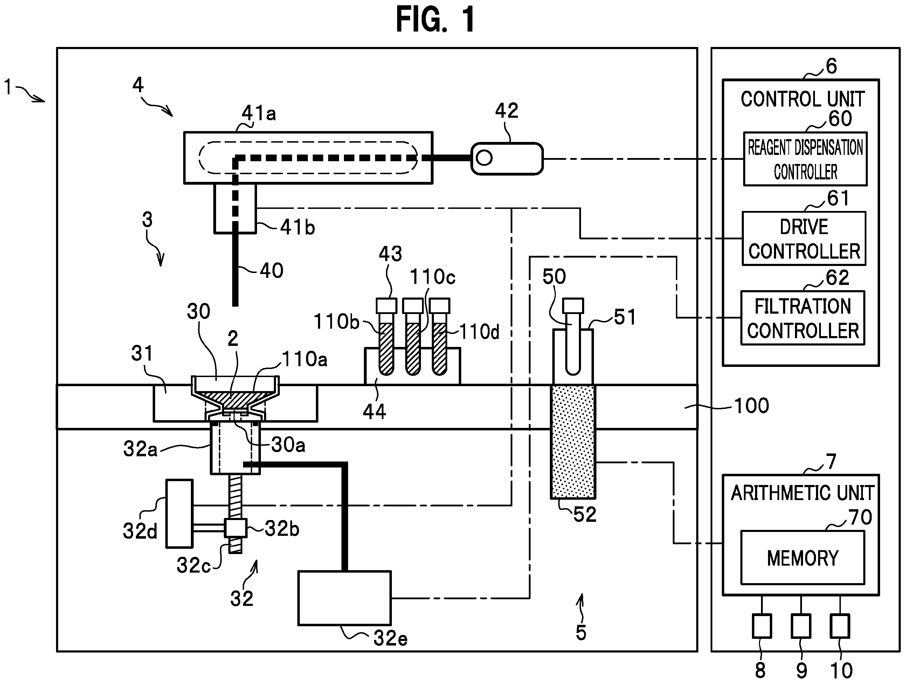

As shown in FIG. 1, a microorganism measuring system 1 is formed from the following multiple components. The microorganism measuring system 1 includes: a filter collection unit 3 configured to collect microorganisms from within a liquid sample 2 onto a filter 30a; a reagent dispenser 4 configured to take and dispense aliquots of reagents to be used in the ATP method; a luminescence measurement unit 5 configured to measure ATP luminescence; a control unit 6 configured to control operations of a pump and a motor, which will be described later; an arithmetic unit 7 configured to do arithmetic for calculating an ATP content in the liquid sample 2, and setting the control reference values, based on measurement results; a data input unit 8 through which data can be inputted when keys on it are manipulated; a printer 9 configured to output the measurement results in print; and a display 10 configured to display progress in measurement steps and the measurement results.

It should be noted that: luminous reaction means in claims 1 and 12 of the present invention corresponds to the filter collection unit 3 and the reagent dispenser 4; optical measurement means in claims 1 and 12 corresponds to the luminescence measurement unit 5; and arithmetic control means in claims 1 and 12 correspond to the arithmetic unit 7.

(Filter Collection Unit)

The filter collection unit 3 collects microorganism by separating the microorganisms from within the liquid sample 2. The filter collection unit 3 includes: a collection container 30 having the filter 30a in it; a collection container holder 31; and a filtration device 32 configured to vacuum-draw contents from within the collection container 30 through the filtration device 32.

The collection container 30 separates microorganism from within the sample 2 which is poured into the collection container 30, and collects ATP by extracting the ATP from the separated microorganisms.

The collection container 30 includes the filter 30a to be set in a bottom portion of a funnel-shaped main body of the collection container 30. The filter 30a of this embodiment is a two-ply filter formed from a hydrophilic filter and a hydrophobic filter, although not illustrated. The hydrophilic filter is placed as the upper of the two plies, while the hydrophobic filter is placed as the lower one of the two plies.

The thus-configured collection container 30 retains the sample 2, which has been poured into the main body, on the filter 30a until the sample 2 is sucked using a below-described suction head 32a of the filtration device 32. In addition, when the sample 2 is sucked using the suction head 32a, the filter 30a is capable of discharging a liquid component of the sample 2 to the suction head 32a via the filter 30a while leaving microorganisms (not illustrated) on the filter 30a.

Incidentally, the collection container 30 is detachably attached to a predetermined position on a process stage 100 provided inside the microorganism measuring system 1.

The filtration device 32 includes: the suction head 32a; a nut 32b, a ball screw shaft 32c and a motor 32d configured to move the suction head 32a upward and downward; and a suction pump 32e configured to vacuum-draw the contents from within the collection container 30 through the suction head 32a.

The suction head 32a is movable upward and downward, as described above. When the contents of the collection container 30 is filtered by the filter 30a, the suction head 32a moves upward, and is thus connected to the collection container 30. Furthermore, when the collection container 30 is attached to or detached from the process stage 100, the suction head 32a moves downward, and is thus disconnected from the collection container 30.

When the suction pump 32e connected to the suction head 32a like this is activated, the liquid component is discharged, as described above, from within the collection container 30 to a wastewater storage tank (not illustrated) of the microorganism measuring system 1 via the filter 30a, the suction head 32a and the suction pump 32e.

Incidentally, the drive and stop of the suction pump 32e, as well as the drive and stop of the motor 32d are controlled by a drive controller 61 and a filtration controller 62 of the control unit 6.

(Reagent Dispenser)

The reagent dispenser 4 includes: a nozzle 40; an X, Y-axis actuator 41a and a Z-axis actuator 41b configured to move the nozzle 40 inside the housing of the microorganism measuring system 1; a pump 42 configured to make the nozzle 40 suck or discharge a predetermined amount of liquid; and reagent tubes 43 configured to store reagents; and a reagent tube holder 44.

The reagent dispenser 4 is configured such that: in a step of, albeit described later, measuring an ATP content in the sample 2, the nozzle 40 takes an aliquot of an ATP eliminating reagent (not illustrated) and an aliquot of an ATP extraction reagent (not illustrated) from their respective reagent tubes 43, and dispenses them into the collection container 30 containing the sample 2; and the nozzle 40 transfers a predetermined amount of obtained reaction liquid 110a to a measurement tube 50 which is one of the components of the luminescence measurement unit 5 (described next). The nozzle 40 further takes an aliquot of an ATP luminescence reagent 110d from its corresponding reagent tube 43, and dispenses it into the measurement tube 50.

Moreover, when, albeit described later, an ATP content in a blank sample liquid 110b is measured, the nozzle 40 takes another aliquot of the ATP eliminating reagent and another aliquot of the ATP extraction reagent from their respective reagent tubes 43, and dispenses them into a blank sample container (not illustrated), and transfers the obtained blank sample liquid 110b to the measurement tube 50. The nozzle 40 further takes another aliquot of the ATP luminescence reagent 110d from its corresponding reagent tube 43, and dispenses it into the measurement tube 50.

In addition, when, albeit described later, an ATP content in an ATP standard reagent 110c is measured, the nozzle 40 takes an aliquot of the ATP standard reagent 110c from its corresponding reagent tube 43, and dispenses it into the measurement tube 50; and takes another aliquot of the ATP luminescence reagent 110d from its corresponding reagent tube 43, and dispenses it into the measurement tube 50. Incidentally, the above-mentioned reaction liquid 110a, blank sample liquid 110b and ATP standard reagent 110c are the liquids for the ATP measurement.

Meanwhile, the three-dimensional movement of the nozzle 40 using the XY-axis actuator 41a and the Z-axis actuator 41b of the reagent dispenser 4 is achieved by predetermined control by the drive controller 61. The collection container 30 and the reagent tubes 43 include sensors (not illustrated) installed in their respective reagent replenishment sections to detect the liquid surfaces. Based on information from the sensors, the position of the nozzle 40 in the Z-axis direction for the reagent dispensation is controlled. Furthermore, the adjustment of the reagent aliquots to be taken and dispensed is achieved by predetermined control by a reagent dispensation controller 60.

(Luminescence Measurement Unit)

The luminescence measurement unit 5 includes: the measurement tube 50 configured to receive the ATP luminescence reagent 110d and one of the reaction liquid 110a, the blank sample liquid 110b and the ATP standard reagent 110c, as well as to make them cause luminous reaction; a measurement tube holder 51; and a photon counting unit 52 including a photomultiplier tube for detecting their luminescence intensities during their luminous reaction.

The photon counting unit 52 outputs detection signals representing the respective ATP luminescence intensities to the arithmetic unit 7. To put it specifically, the photon counting unit 52 outputs the detection signal representing the "ATP luminescence intensity of the reaction liquid," the detection signal representing the "ATP luminescence intensity of the blank sample liquid, " and the detection signal representing the "ATP luminescence intensity of the ATP standard reagent."

(Arithmetic Unit)

Based on the detection signal representing the "ATP luminescence intensity of the reaction liquid" and the detection signal representing the "ATP luminescence intensity of the blank sample liquid" outputted from the photon counting unit 52, the arithmetic unit 7 does arithmetic on differential luminescence intensity between the two ATP luminescence intensities. Furthermore, based on the differential luminescence intensity and the "ATP luminescence intensity of the ATP standard reagent," the arithmetic unit 7 obtains the ATP content in the microorganisms in the sample 2 by doing arithmetic.

Measurement results are associated with information on the sample 2 which is inputted from the data input unit 8 during the ATP measurement of the sample. The thus-associated measurement results are stored into a memory 70 in the arithmetic unit 7. It should be noted that in a step in claims of repeating CFU counting in a normal condition, the information on the sample further includes the result of the CFU counting.

The memory 70 in the arithmetic unit 7, meanwhile, stores condition values for the microbiological control, such as tolerances for false positives and false negatives. Using the ATP measurement results and the condition values, the arithmetic unit 7 performs a series of calculations to construct a probability model for ATP measured values of the measurement object, and calculates the control reference values. Detailed descriptions will be later provided for these calculations.

<Operation of Microorganism Measuring System and Principle of ATP Assay>

Next, descriptions will be provided for operation of the microorganism measuring system and a principle of an ATP assay.

The microorganism measuring system 1 works as follows. The collection container 30 is placed on the process stage 100. Subsequently, the sample 2 as the examination object is poured into the collection container 30. Thereafter, once an activation switch (not illustrated) is turned on, the control unit 6 starts to perform the following procedure.

The suction head 32a moves upward, and is connected to the collection container 30. Subsequently, the suction pump 32e is activated, and starts to filter the contents (sample 2) of the collection container 30. In this filtering step, the microorganisms are collected onto the filter 30a, while the liquid component of the sample 2 is discharged to the suction head 32a. In that case, most of ATP existing outside the cells of the microorganisms included in the liquid component of the sample 2, and substances which would otherwise obstruct the ensuing luminous reaction of the ATP are discharged with the liquid component.

Subsequently, after the filtration of the contents of the collection container 30 is completed, a buffer liquid is poured into the collection container 30, and the suction pump 32e is activated again to filter the contents of the collection container 30.

Thereby, microorganisms remaining on the inner surface and the like of the collection container 30 are also collected onto the filter 30a. It is desirable that sterilized water or the like including no ATP be used as the buffer liquid.

In the next step, an aliquot of the ATP eliminating reagent is dispensed into the collection container 30.

The ATP eliminating reagent is stored in its corresponding reagent tube 43 (albeit not illustrated). The nozzle 40 of the reagent dispenser 4 takes a predetermined aliquot of the ATP eliminating reagent from the reagent tube 43, and dispenses it into the collection container 30.

ATP existing outside the cells of the microorganisms is more securely eliminated by dispensing the aliquot of the ATP eliminating reagent. ATPases can be cited as examples of the ATP eliminating reagent.

Subsequently, the ATP extraction reagent (albeit not illustrated) is stored in its corresponding reagent tube 43. The nozzle 40 of the reagent dispenser 4 takes a predetermined aliquot of the ATP eliminating reagent from the reagent tube 43, and dispenses it into the collection container 30.

ATP included in the microorganisms is extracted by dispensing the aliquot of the ATP extraction reagent. Thereby, the reaction liquid 110a is produced inside the collection container 30.

Preferably usable examples of the ATP extraction reagent include surfactants, liquid mixtures of ethanol and ammonia, ethanol buffer solutions, methanol buffer solutions, trichloroacetic acid buffer solutions, and Tris buffer solutions.

Thereafter, the nozzle 40 of the reagent dispenser 4 takes an aliquot of the reaction liquid 110a from the collection container 30, and dispenses it into the measurement tube 50 of the luminescence measurement unit 5.

Furthermore, the nozzle 40 of the reagent dispenser 4 takes an aliquot of the ATP luminescence reagent 110d from the corresponding reagent tube 43 which stores the ATP luminescence reagent 110d, and dispenses it into the measurement tube 50 of the luminescence measurement unit 5.

A luciferase-luciferin reagent can be cited as an example of the ATP luminescence reagent 110d.

By this, ATP in the reaction liquid 110a and the ATP luminescence reagent 110d react on each other in the measurement tube 50, and accordingly cause luminescence.

Next, the arithmetic unit 7 digitally processes the detection signal which the photon counting unit 52 outputs by detecting the ATP luminescence intensity, and thereby measures the luminescence intensity based on the single photon counting method.

Subsequently, a value (datum) representing the luminescence intensity is temporarily stored in the memory 70.

Thereafter, the nozzle 40 takes an aliquot of the ATP eliminating reagent from the corresponding reagent tube 43 which stores the ATP eliminating reagent, and dispenses it into the blank sample container (not illustrated) similarly placed in a reagent tube 43.

Furthermore, the nozzle 40 takes an aliquot of the ATP extraction reagent from the corresponding reagent tube 43 which stores the ATP extraction reagent, and dispenses it into the blank sample container.

By dispensing the aliquot of the ATP extraction reagent, the blank sample liquid 110b which includes reagent- and container-derived ATP which will not be measured is prepared inside the blank sample container.

Subsequently, the measurement tube 50 is replaced with a new one. After that, the nozzle 40 takes an aliquot of the blank sample liquid 110b from the blank sample container, and dispenses it into the measurement tube 50 of the luminescence measurement unit 5.

Furthermore, the nozzle 40 takes an aliquot of the ATP luminescence reagent 110d from the corresponding reagent tube 43 which stores the ATP luminescence reagent 110d, and dispenses it into the measurement tube 50.

By this, ATP in the blank sample liquid 110b and the ATP luminescence reagent 110d react on each other in the measurement tube 50, and accordingly cause luminescence.

Next, the arithmetic unit 7 digitally processes the detection signal which the photon counting unit 52 outputs by detecting the ATP luminescence intensity, and thereby measures the luminescence intensity based on the single photon counting method.

Subsequently, a value (datum) representing the luminescence intensity is temporarily stored in the memory 70.

Subsequently, the measurement tube 50 is replaced with a new one. After that, the nozzle 40 takes an aliquot of the ATP standard reagent 110c from the corresponding reagent tube 43 which stores the ATP standard reagent 110c, and dispenses it into the measurement tube 50.

Furthermore, the nozzle 40 takes an aliquot of the ATP luminescence reagent 110d from the corresponding reagent tube 43 which stores the ATP luminescence reagent 110d, and dispenses it into the measurement tube 50.

By this, ATP in the ATP standard reagent 110c and the ATP luminescence reagent 110d react on each other in the measurement tube 50, and accordingly cause luminescence.

Next, the arithmetic unit 7 digitally processes the detection signal which the photon counting unit 52 outputs by detecting the ATP luminescence intensity, and thereby measures the luminescence intensity based on the single photon counting method.

Subsequently, a value (datum) representing the luminescence intensity is temporarily stored in the memory 70.

Thereafter, the arithmetic unit 7 does the arithmetic on the differential luminescence intensity between the luminescence intensity of the sample 2 and the luminescence intensity of the blank sample. Furthermore, based on the luminescence intensity of the ATP standard reagent, the arithmetic unit 7 does arithmetic on an ATP content (amol) corresponding to the differential luminescence intensity, displays the measurement results on the display 10, and outputs the measurement results from the printer 9. With these, the microorganism measuring system 1 completes the series of ATP measurement steps.

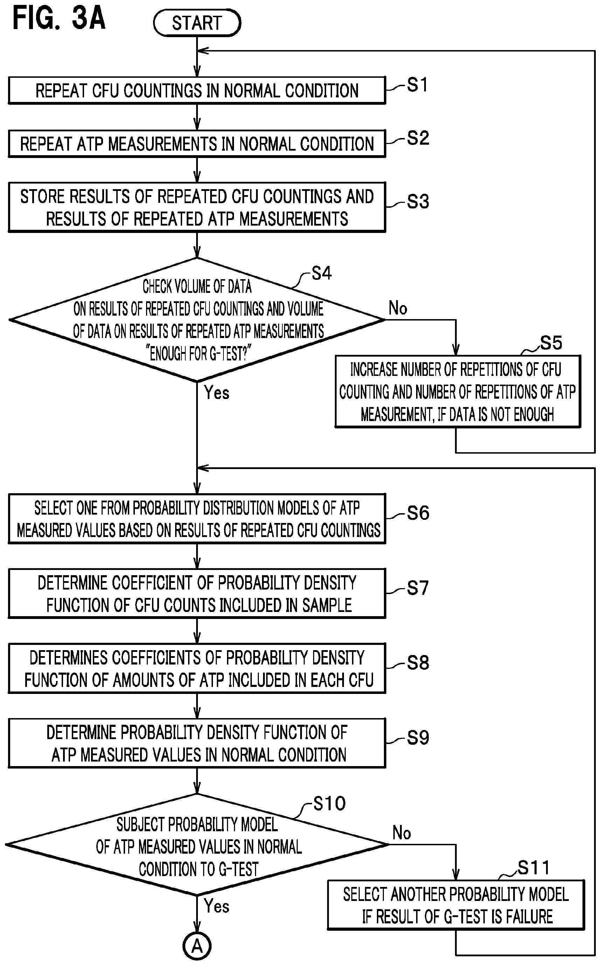

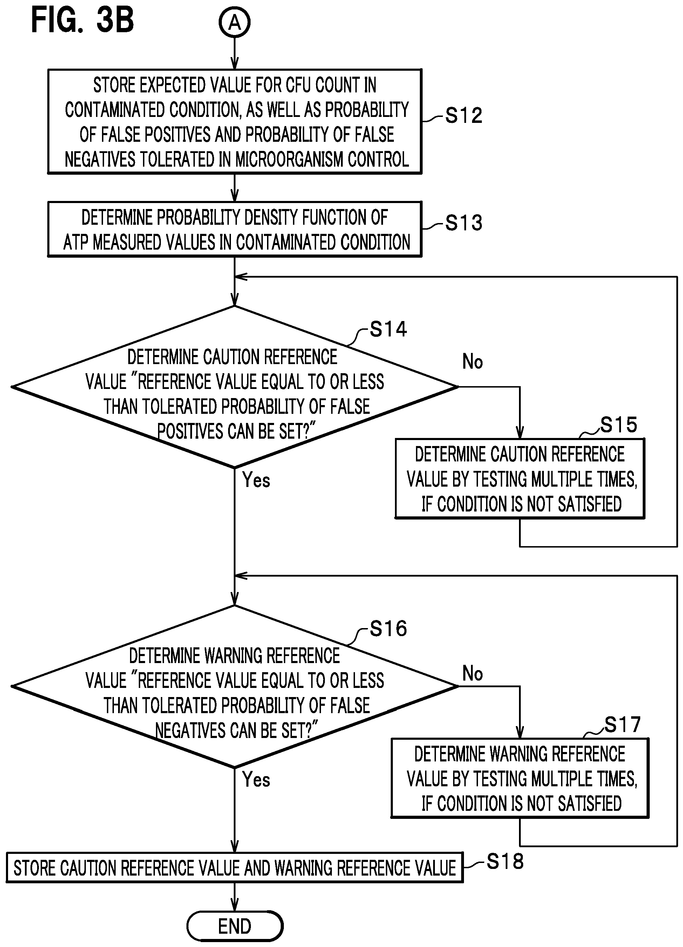

The above-described series of ATP measurement steps corresponds to step S2 in FIGS. 3A and 3B which show a flowchart illustrating an outline of how to set the control reference values in the microorganism measuring system 1 of the embodiment of the present invention.

It should be noted that before the series of ATP measurement steps, information on the sample 2, inclusive of a type of measurement object, a sampling position and a lot of the reagent in use, is stored into the memory 70 using the data input unit 8.

Furthermore, as described above, the information stored in the memory 70 in association with the ATP measurement results includes the result of the CFU counting (counting manually or mechanically) to be performed together with the ATP measurement.

The CFU counting step corresponds to step S1 in FIGS. 3A and 3B which show the flowchart illustrating the outline of how to set the control reference values in the microorganism measuring system 1 of the embodiment of the present invention (hereinafter omitting the phrase "in FIGS. 3A and 3B" from time to time). Independently of the ATP measurement step (Step S2), the result of the CFU counting is stored into the memory 70.

<Probability Distribution Model for ATP Measured Values>

This section will describe probability density functions which serve as bases of probability distribution models for the ATP measured values in this embodiment.

The probability distribution model to be used is one which is selected mainly from the following two models depending on what condition the measurement object is in.

(Compound Poisson Distribution Model)

The model corresponds to an environment where the number of bacteria in the sample is small.

The ATP measured values S represent the sum of ATP contents in collected bacteria, and are expressed with Equations (1) given below:

$$S = X_1 + X_2 + \cdots + X_N = \sum_{i=1}^{N} X_i \, (N \geq 1)$$

$$S = X^0 \, (N = 0) \qquad \cdots (1)$$

where N denotes the number of collected bacteria, $X_i$ denotes an ATP content per bacterium, and $X^0$ denotes a measured

value of an ATP content in the environment from which no bacteria are collected. N, $X_i$ and $X^0$ are stochastic variables under a certain a probability distribution.

(1) Distribution of Number N of Collected Bacteria

The number N of collected bacteria is considered to obey a Poisson distribution. A probability density function $f_N(k)$ is expressed with Equations (2) given below:

$$N \sim Po(\lambda)$$

$$f_N(k) = \frac{\lambda^k e^{-\lambda}}{k!} \quad \cdots (2)$$

where $\lambda$ denotes a parameter of the probability model. $\lambda$ is dependent on an expected value for the number of bacteria collected in the environment.

(2) Distribution of Amounts of ATP

One may consider that the ATP contents $X_i$ in the bacteria respectively obey certain distributions which are independent from one another. The distribution of $X_i$ varies depending on the types of the bacteria, and the activity states of the bacteria. The distribution further includes variations stemming from the measuring system, such as variations in the reagent reactions. This method approximates $X_i$ using a gamma distribution. A probability density function $f_x(x)$ is expressed with

$$X_i \sim \Gamma(\eta, \kappa)$$

$$f_x(x) = \frac{x^{\eta-1} e^{-x/\kappa}}{\Gamma(\eta)\kappa^\eta}$$

$$\eta = \frac{\mu^2}{\sigma^2}$$

$$\kappa = \frac{\sigma^2}{\mu}$$

where $\mu$ and $\sigma$ are parameters of the probability model. $\mu$ and $\sigma$ respectively denote the mean and standard deviation of the ATP contents per bacterium. $\mu$ and $\sigma$ vary depending on types of collected bacteria, and the measurement environment.

Suppose what the ATP content $X^k$ is when k bacteria are collected. The ATP content $X^k$ is expressed with

$$X^k = X_1 + X_2 + \cdots + X_k$$

Because of the reproductive property of the gamma distribution, $X^k$ obeys a gamma distribution represented by Equations (3) given below:

$$X^k \sim \Gamma(k\eta, \kappa)$$

$$f_X^k(x) = \frac{x^{k\eta-1}e^{-x/\kappa}}{\Gamma(k\eta) \cdot (\kappa)^{k\eta}} (k \geq 1) \quad \cdots (3)$$

(3) ATP Measured Value $X^0$ When No Bacteria Are Collected

$X^0$ is considered to correspond to a variation in blank measurement values. $X^0$ is approximated using a normal distribution represented by

$$X^0 \sim N(\mu_b, \sigma_b^2)$$

$$f_X^0(x) = \frac{1}{\sqrt{2\pi\sigma_b^2}} \exp(-\frac{(x-\mu_b)^2}{2\sigma_b^2}) \quad \cdots (4)$$

where $\mu_b$ and $\sigma_b$ respectively denote the mean and the standard deviation obtained from the blank measurement using a Biomaytector (a registered trademark. a high-sensitive ATP measurement apparatus made by Hitachi, Ltd.). $\mu_b$ and $\sigma_b$ are determined according to the apparatus specifications.

(4) Probability Density Function of ATP Measured Values

Using Equations (2), (3) and (4), a probability density function $f_s(x)$ of the ATP measured values S can be expressed with Equation (5) given below:

$$f_S(x) = \sum_{k=0}^{\infty} f_N(k) f_X^k(x) \quad \cdots (5)$$

It should be noted that the actual calculation is terminated when the number $k_{max}$ of bacteria considered to cause no practical problem is reached.

(Normal Distribution Model)

In a case of an environment where the mean number of collected bacteria is large, one may consider that the distribution of the ATP measured values can be almost approximated using a normal distribution. With the consistency between the parameters taken into consideration, the ATP measured values S are approximated from the mean $\mu s = \lambda\mu$, and the variance $\sigma_s^2 = (\mu^2 + \sigma^2)$ in the compound Poisson distribution, using Equations (6) given below:

$$S \sim N(\lambda\mu, \lambda(\mu^2 + \sigma^2))$$

$$f_S(x) = \frac{1}{\sqrt{2\pi\lambda(\mu^2+\sigma^2)}} \exp(-\frac{(x-\lambda\mu)^2}{2\lambda(\mu^2+\sigma^2)}) \quad \cdots (6)$$

where: $\lambda$ is a parameter representing the expected value for the number of bacteria collected; and $\mu$ and $\sigma$ are parameters determined depending on the measurement environment, including the types of collected bacteria.

<Parameter Setting for Probability Distribution Model for ATP Measured Values>

The microorganism measuring system 1 of the embodiment requires the parameters for the probability models to be estimated before actually starting its operation. For this reason, before the actual operation, the parameters for the normal condition are estimated by repeating the measurement in the normal condition. Data needed for the estimation are the number of collected bacteria measured using a culture method, and ATP contents measured by the microorganism measuring system 1.

(Compound Poisson Distribution Model)

Let c be the mean of the numbers of collected bacteria in the respective prior measurements repeated before the actual operation, $q_t$ be an amount of sample collected for the culture method, and $q_b$ be an amount of sample collected for the biomaytector. The parameter $\lambda$ for the Poisson distribution is obtained using Equation (7) given below:

$$\lambda = \bar{c}\frac{q_b}{q_t} \quad \cdots (7)$$

When values obtained by repeatedly measuring the ATP content are $x_1$, $x_2$, ..., $x_m$, the parameters $\mu$ and $\sigma$ for the distribution of the ATP contents in the bacteria are adjusted such that the log-likelihood function 1 $(\lambda, \mu, \sigma)$ expressed with Equation (8) given below achieves its largest value:

$$l(\lambda, \mu, \sigma) = \log(\prod_{i=1}^{m} f_S(x_i : \lambda, \mu, \sigma))$$

$$= \sum_{i=1}^{m} \log(f_S(x_i : \lambda, \mu, \sigma)) \quad \cdots (8)$$

(Normal Distribution Model)

Let c be the mean of the numbers of the collected bacteria in the respective prior measurements, $q_t$ be an amount of sample collected for the culture method, and $q_b$ be an amount of sample collected for the biomaytector. In addition, let $\bar{x}$ be the mean of ATP contents in the respective repeated measurements, and $u^2$ be the unbiased variance. Equations (9) given below yield $\lambda$, $\mu$ and $\sigma$, respectively:

$$\lambda = \bar{c}\frac{\overline{q_b}}{q_t}$$

$$\mu = \frac{\bar{x}}{\lambda}$$

$$\sigma = \sqrt{\frac{u^2}{\lambda} - \frac{\bar{x}^2}{\lambda^2}} \quad \cdots (9)$$

\<Verification of Probability Models and Number of Prior Measurements>

(Verification of Probability Models)

The estimated probability models are subjected to G-tests, respectively. Both the distribution of the numbers of bacteria (the Poisson distribution) and the distribution of ATP (the compound Poisson distribution) are subjected to the respective tests. The distribution of the numbers of bacteria is subjected to a G-test with the number of classes being 4 and the number of degrees of freedom being 2, since the number of estimated values from the sample is 1($\lambda$). The distribution of ATP is subjected to a G-test with the number of classes being 4 and the number of degrees of freedom being 1, since the number of estimated values from the sample is 2($\mu$, $\sigma$).

It should be noted that in each G-test, the relationship among the number of classes, the number of degrees of freedom and the number of estimated values satisfies

```
Number of Degrees of Freedom = Number of Classes-1-Number of Estimated Values.
```

In addition, since the number of degrees of freedom needs to be equal to or greater than 1, the number of classes is set such that the number of degrees of freedom becomes equal to or greater than 1.

For the distribution of the numbers of bacteria, the number of classes may be set at 3, but needs to be set at 4 because there is a possibility that another probability model with two parameters (two estimated values) may be employed.

Generally speaking, for the G-test, it is desirable that the expected value for the frequency in each class be equal to or greater than 5. For this reason, the number of prior measurements needs to be set such that the expected value for the frequency value in each class becomes equal to or greater than 5.

Based on the finite variance credibility theory,

$$n_F = \left(\frac{y_{\varepsilon/2}}{\alpha}\right)^2 \left\{1 + \left(\frac{\sigma}{\mu}\right)^2\right\}$$

yields the total $n_F$ of the collected bacteria with the mean $\bar{x}$ of the measured values falling within a range of E(s) minus $\alpha$E(s) toE(s) plus $\alpha$E(s) with a probability of (1-$\varepsilon$), where E (s) denotes the true mean.

In the above equation, $y_{\varepsilon/2}$ denotes the upper $\varepsilon/2$ point in the standard normal distribution. $\mu$ and $\sigma$ respectively denote the mean value and the standard deviation of the ATP contents per bacterium. It is desirable that the prior measurements be repeated until the total $n_F$ of the collected bacteria comes to satisfy a predetermined accuracy. The determinant of the accuracy is the number of collected bacteria. For this reason, a cleaner environment where the number of collected bacteria is smaller requires a larger number of prior measurements, while a less clean environment where the number of collected bacteria is larger requires only a smaller number of prior measurements.

<Procedure for Setting Control Reference Values for ATP Measured Value>

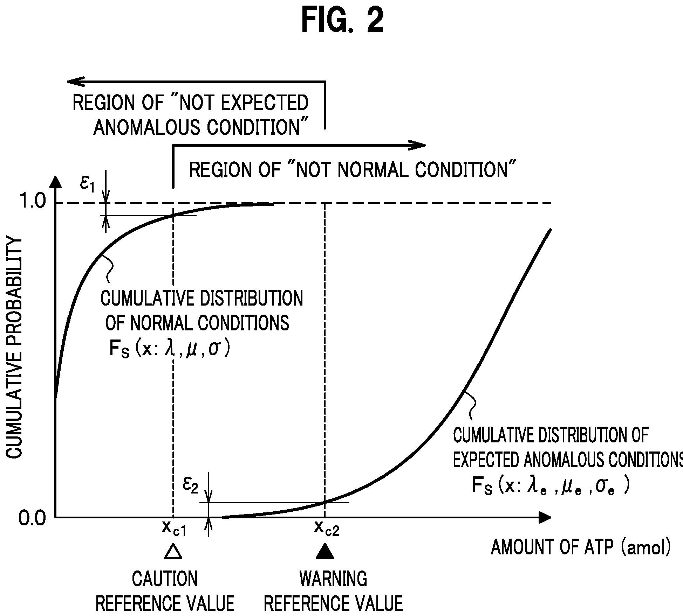

FIG. 2 is an explanatory diagram showing a relationship among false positive, false negative and control reference values in the microorganism measuring system 1 of the embodiment of the present invention.

(Setting Alert reference value)

Using the measured values S, the normal condition (=a null hypothesis) is subjected to a right-tailed test at a significance level of $\varepsilon_1$. If the normal condition is rejected by the test, one may consider that the condition is not normal at the significance level of $\varepsilon_1$. If the normal condition is accepted by the test, it is impossible to assert that the condition is "not the normal condition."

A range for rejecting the normal condition is determined as $S > x_{c1}$, where $x_{c1}$ is obtained from Equations (10) :

$$P(S > x_{c1}) \leq \varepsilon_1$$

$$F_S(x_{c1}) = \int_0^{x_{c1}} f_S(x)dx = 1 - \varepsilon_1 \quad \cdots (10)$$

$x_{c1}$ is used as the alert reference value.

(Setting Action reference value)

A test is carried out using $\lambda_e$, $\mu_e$ and $\sigma_e$ as parameters for the probability distribution model for the ATP contents in a contaminated condition.

$\lambda_e$ denotes an expected value for the number of bacteria in the contaminated condition. The number of bacteria used a reference in the culture method may be employed as $\lambda_e$. The parameter values used for the normal condition are similarly employed as $\mu_e$ and $\sigma_e$ if the bacterial condition in the contaminated condition is not different from that in the normal condition. Otherwise, the general mean of the ATP contents and the general standard deviation may be employed as $\mu_e$ and $\sigma_e$.

Using the measured value S, the expected contaminated condition (=a null hypothesis) is subjected to a left-tailed test at a significance level of $\varepsilon_2$. If the contaminated condition is rejected by the test, one may consider that the condition is not contaminated at the significance level of $\varepsilon_2$. If the contaminated condition is accepted by the test, it is impossible to assert that the condition is "not the contaminated condition (expected anomalous condition)."

A range for rejecting the contaminated condition is determined as $S < x_{c2}$, where $x_{c2}$ is obtained from Equations (11) :

$$P(S < x_{c2}) \leq \varepsilon_2$$

$$F_S(x_{c2} : \lambda_e, \mu_e, \sigma_e) = \int_0^{x_{c2}} f_S(x : \lambda_e, \mu_e, \sigma_e)dx = \varepsilon_2$$

$$\dots (11)$$

$x_{c2}$ is used as the action reference value.

It should be noted that statistically-general numbers, such as 0.01 (1%) and 0.05 (5%) may be used as the significance levels of $\varepsilon_1$ and $\varepsilon_2$. However, it is desirable that $\varepsilon_1$ and $\varepsilon_2$ be set at appropriate values by the administrator depending on the operational needs, because: $\varepsilon_1$ represents a probability of false positives with which an uncontaminated condition is determined as being contaminated; and $\varepsilon_2$ represents a probability of false negatives with which a contaminated condition is determined as being uncontaminated (for which detailed descriptions will be provided later).

<Procedure for Setting Control Reference Values for ATP Measured Value>

Referring to FIGS. 3A and 3B, descriptions will be provided for a procedure for setting the control reference values for the ATP measured value. FIGS. 3A and 3B provide a flowchart showing an outline of how to set the control reference values in the microorganism measuring system of the embodiment of the present invention.

First of all, under the condition where the measurement object is normal, the CFU counting is repeated (in step S1), and the ATP measurement is repeated (in step S1). Prior to the ATP measurement, the information on the sample 2, inclusive of the sampling position and the lot of the reagent in use, is stored into the memory 70 using the data input unit 8.

The result of the CFU counting is stored into the memory 70 of the microorganism measuring system 1 using the data input unit 8. Meanwhile, the ATP measurements are performed by the microorganism measuring system 1, and the ATP measurement results are stored into the memory 70 (in step S3).

It is desirable that the repeated CFU counting and the repeated ATP measurement be performed at the same time. However, in a case where data are collected on the same measurement object under the normal condition, the repeated CFU counting and the repeated ATP measurement do not have to be performed at the same time.

Thereafter, the arithmetic unit 7 tests whether or not the volume of data on the results of the repeated CFU countings stored in the memory 70, and the volume of data on the results of the repeated ATP measurements stored in the memory 70 are large enough for the G-test (in step S4). To put it specifically, this determination is made based on whether the expected frequency value for each class is not less than 5 as described above.

If the determination in step S4 is NO, the procedure increases the number of repetitions of the CFU counting and the number of repetitions of the ATP measurement (in step S5) by returning to the repeating of the CFU counting and the repeating of the ATP measurement.

If the determination in step S4 is YES, the arithmetic unit 7 selects a suitable one from the probability distribution models for the ATP measured values based on the result of the repeated CFU counting (in step S6). To put it specifically, if the mean number of collected bacteria in the repeated CFU counting is equal to or less than 100 CFUs, the arithmetic unit 7 selects the compound Poisson distribution model. If the mean number of collected bacteria in the repeated CFU counting is greater than 100 CFUs, the arithmetic unit 7 selects the normal distribution model. The following section will describe a flow with the assumption that the compound Poisson Model is selected.

Subsequently, using Equation (7), the arithmetic unit 7 determines the coefficient $\lambda$ in the probability density function (Equations (2)) for the CFU counts included in the sample (in step S7). In addition, using Equation (8), the arithmetic unit 7 further determines the coefficients $\mu$ and $\sigma$ in the probability distribution function (Equations (3)) of the ATP contents per CFU. Incidentally, step S7 and step S8 may be performed in a reverse order or in parallel.

Furthermore, the arithmetic unit 7 determines the probability density function of the ATP measured values in the normal condition by substituting the determined coefficients into Equation (5) (step S9).

Subsequently, the arithmetic unit 7 subjects the probability model for the ATP measured values in the normal condition, which is determined in step S9, to the G-test (in step S10). As described above, both the distribution of the CFU counts included in the sample (the Poisson distribution) and the distribution of the ATP measured values (the compound Poisson distribution) are subjected to the respective tests. The G-test on the distribution of the CFU counts is performed with the number of classes being 4 and the number of degrees of freedom being 2. The G-test on the distribution of the ATP measured values is performed with the number of classes being 4 and the number of degrees of freedom being 1.

If the determination in step S10 is NO, the arithmetic unit 7 determines that the probability distribution of the ATP measured values of the measurement object cannot be approximated by the selected probability model, and selects another probability model (in steps S11 and S6). In this case, another probability model other than the above-described probability models is used. However, one may consider that such a case rarely happens. For this reason, detailed description for another probability model will be omitted.

If the determination in step S10 is YES, the procedure proceeds to a step of constructing the probability model for the ATP measured values in the contaminated condition. First of all, the expected value for the CFU count in the contaminated condition, as well as the probability of false positives and the probability of false negatives tolerated in the microbiological control are stored into the memory 70 using the data input unit 8 (in step S12).

In this respect, the expected value for the CFU count in the contaminated condition means a value for the CFU count expected when the measurement object is contaminated. A conventional control reference value for the CFU count, the CFU count in a contaminated condition in a contamination case in the past, or the like may be used as the expected value for the CFU count.

Furthermore, the probability of false positives and the probability of false negatives tolerated in the microbiological control define the accuracy of the microbiological control, and may be set depending on types of measurement objects, and examination purposes. For example, a higher accuracy of the microbiological control may be set for an object having a higher contamination risk, such as a product itself under an inspection or an environment to which products are directly exposed.

Thereafter, the arithmetic unit 7 determines the probability density function of the ATP measured values in the contaminated condition using: the expected value for the CFU count in the contaminated condition inputted in step S12; and the probability density function of the ATP measured values in the normal condition determined in step S9 (in step S13).

To put it specifically, the expected value for the CFU count in the contaminated condition is substituted into $\lambda_e$ of the coefficients $\lambda_e$, $\mu_e$ and $\sigma_e$ of the probability density function for the contaminated condition. On the other hand, it is desirable that $\mu_e$ and $\sigma_e$ be set as follows, depending on what the measurement object is.

A first method is employed as follows. When the measurement object is a closed environment, or an environment whose characteristic property such as pH has an effect on the growth of microorganisms, values which are the same as those in the probability density function of the ATP measured values in the normal condition are substituted into $\mu_e$ and $\sigma_e$.

This is because it is considered that: types of microorganisms detected in the above-mentioned measurement object are highly likely to be limited to a certain extent; and the numbers of microorganisms increase while the types of bacteria and their proportions are not different between the above-mentioned measurement object and the normal condition.

On the other hand, there is high likelihood that the types of microorganisms in the measurement object and their proportions are largely different between the normal condition and the contaminated condition, since like in the general ecological system, the types of microorganisms and their proportions are different from one environment to another, and do not remain unchanged even in the same environment, as described above.

With this taken into consideration, a second method is employed as follows. The mean ATP content and the standard deviation obtained using maker bacterial strains or the like in advance are substituted into $\mu_e$ and $\sigma_e$.

Subsequently, in step S14, the arithmetic unit 7 determines the alert reference value. To put it specifically, using Equation (10), the arithmetic unit 7 determines the alert reference value at which the tolerated probability of false positives is equal to or less than the probability inputted in step S12 (in step S14).

If unable to determine the alert reference value which satisfies the condition (if NO in step S14), the arithmetic unit 7 determines the alert reference value which satisfies the condition by performing measurements and tests multiple times (in steps S15 and S14). To put it specifically, in a case where, for example, the measurement is performed at intervals of one hour, the determination of the alert reference value is achieved by using not only the result of the measurement at a particular point of time, but also the results of the multiple measurements at intervals of one hour (similarly in step S17).

After that, the arithmetic unit 7 determines the action reference value. To put it specifically, using Equation (11), the arithmetic unit 7 determines the action reference value at which the tolerated probability of false negatives is equal to or less than the probability inputted in step S12 (in step S16) .

If unable to determine the action reference value which satisfies the condition (if NO in step S16), the arithmetic unit 7 determines the action reference value which satisfies the condition by performing measurements and tests multiple times (in step S17).

The thus-determined alert reference value and action reference value are stored into the memory 70 (in step S18). In the microbiological control using the microorganism measuring system 1, each time the ATP measurement is performed, the arithmetic unit 7 checks whether or not the result of the ATP measurement deviates from these reference values by referring to the reference values. This will be hereinbelow described in details.

It should be noted that although in the embodiment, the storing of the values in step S12 comes after the G-test of the probability models for the ATP measured values in the normal condition (in step S10), the timing for storing the values is not limited to this example. The values may be stored at other timing as long as the values are stored before steps S13, S14 and S16 for which the values are needed.

<Operational Method Using Control Reference Values for ATP Measured Values>

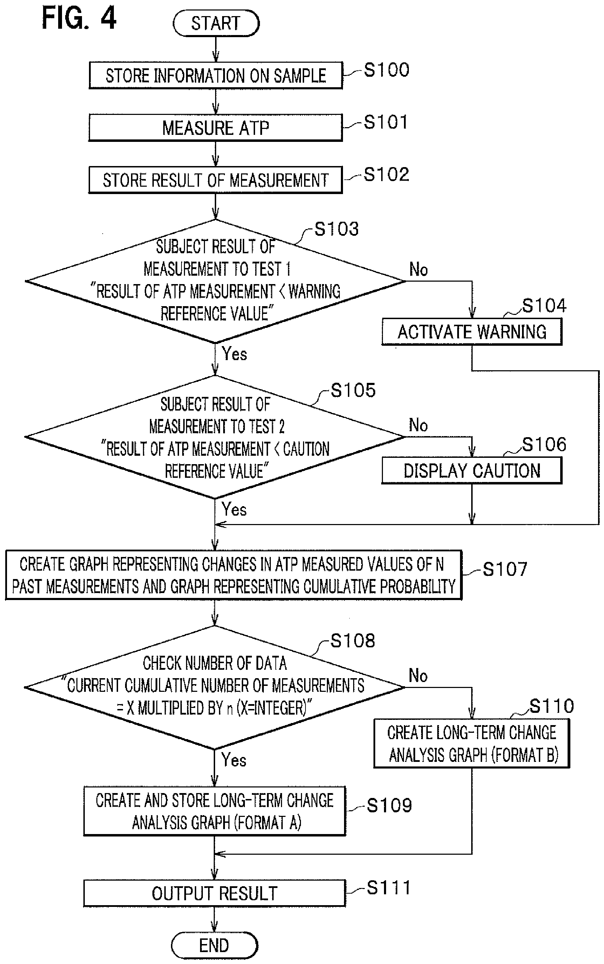

Descriptions will be hereinbelow described for how to actually perform the microbiological control using the control reference values for the ATP measured values, referring to FIGS. 4, 5A, 5B, 6 and 7 as needed.

FIG. 4 is a flowchart showing an outline of how to operate the microorganism measuring system 1 of the embodiment of the present invention.

To begin with, the information on the sample 2, inclusive of the type of measurement objects, the sampling position and the lot of the reagent in use, is stored into the memory 70 using the data input unit 8 (in step S100). Subsequently, the ATP measurement is performed on the sample 2 (in step S101). The results of the measurement are stored into the memory 70 (in step S102). In this step, the results of the measurement are stored by being classified according to the types of measurement objects based on the information on the types of measurement objects and the sampling position inputted in step S100.

Subsequently, the arithmetic unit 7 subjects the results of the measurement to a test 1. The test 1 determines whether or not each result of the measurement is less than the alert reference value (in step S103).

If the determination in step S103 is NO, a caution is issued (in step S104). Specific citable examples of the issuing of the caution include a caution message to be displayed on the screen for displaying the result of the measurement in the display 10, and on a report on the result of the measurement outputted from the printer 9 (the caution may be issued in step S111 described below). Otherwise, the issuing of the caution may be achieved by: using a caution buzzer configured to be activated when triggered by the determination; informing an administrator in a remote place of the result of the measurement by outputting the result of the measurement on-line; or applying an emergency stop to the production line.

If the determination in step S103 is YES, the arithmetic unit 7 immediately subjects the results of the measurement to a test 2. The test 2 determines whether or not each result of the measurement is less than the action reference value (in step S105).

If the determination in step S105 is NO, a warning is displayed (in step S106). Specific citable examples of the displaying of the warning include a warning message to be displayed on the screen for displaying the result of the measurement in the display 10, and on a report on the result of the measurement outputted from the printer 9 (the caution may be issued in step S111 described below).

Subsequently, the arithmetic unit 7 creates graphs. To begin with, based on the information on the types of measurement objects and the sampling position inputted in step S100, the arithmetic unit 7 creates: a graph representing changes in the results of n past measurements of the same measurement object as the sample 2; and cumulative probability graphs (in step S107).

It should be noted that the value n can be freely set in advance using the data input unit 8.

Thereafter, the arithmetic unit 7 checks the number of data. In this respect, the arithmetic unit 7 determines whether or not the cumulative number of measurements at this time can be represented by X multiplied by n (where X is an integer), or is a multiple of n (in step S108). If the determination in step S108 is YES, the cumulative probability graphs created in step S107 is stored, as a long-term change analysis graph (Format A), into the memory 70 (in step S109).

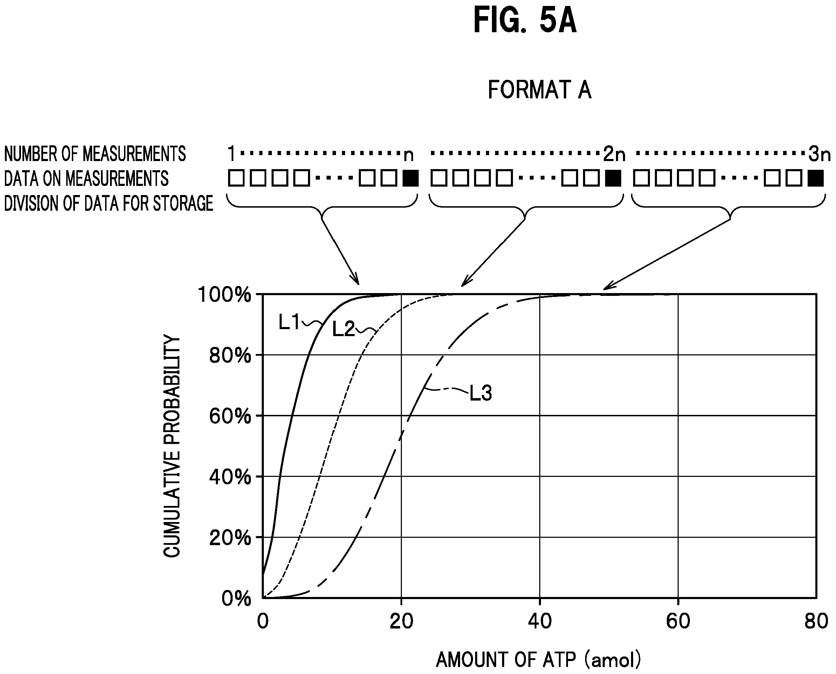

FIG. 5A is a schematic explanatory diagram for explaining graphs and graph data classifications in Format A. The long-term change analysis graph is a graph in which the cumulative probability graphs (L1 to L3) respectively created for the results of the n past measurements of the same measurement object are superposed on one another. When visually reviewing how the cumulative probability graphs are superposed on one another, and what shapes the cumulative probability graphs take on, the long-term change analysis graph makes it possible to check whether or not the probability distribution of the ATP measured values in the measurement object has changed since the facility returns from a break, or due to factors such as seasonal changes.

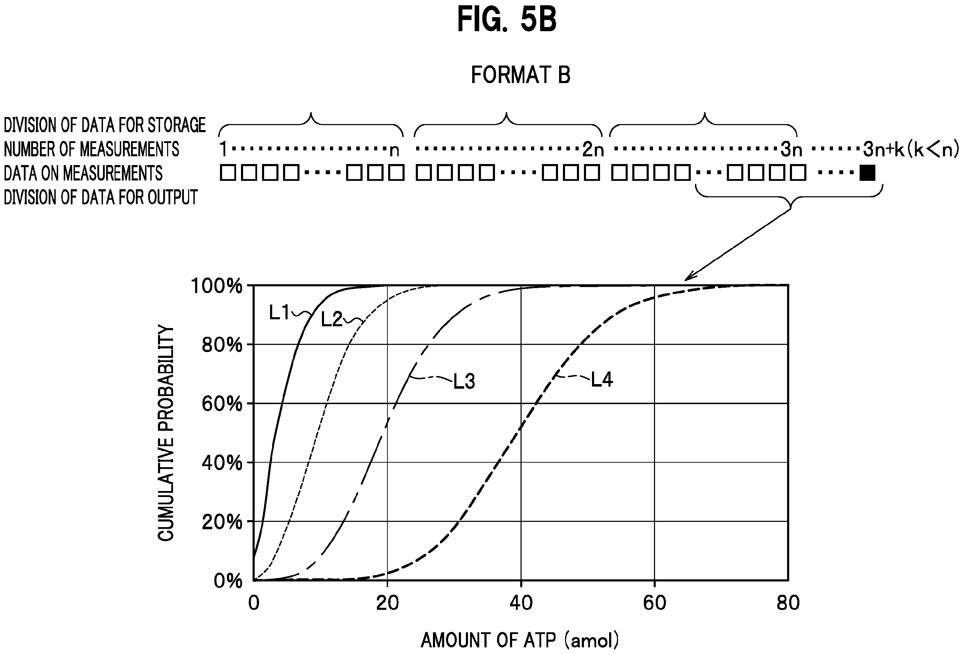

FIG. 5B is a schematic explanatory diagram for explaining graphs and graph data classifications in Format B. If the determination in step S108 is NO, the cumulative probability graphs created in step S107 are not stored into the memory 70. For the purpose of outputting the result, a graph in Format B is created in which, represented by dashed lines and the like, the cumulative probability graphs (L1 to L4) created in step S107 are superposed on the latest long-term change analysis graph created most recently (in step S110).

For each group of n data which is set in advance, the long-term change analysis graph is created by superposing the cumulative probability graphs, as described above.

It should be noted that a first measurement with which the counting of the cumulative number of measurements starts can be freely selected using the data input unit 8. To put it specifically, a first measurement in a series of repeated measurements, a measurement after the facility returns from a break, a measurement after a long-term suspension of the production line, and the like may be set as the first measurement with which the counting of the cumulative number of measurements starts.

Once the two tests on the result of the measurement (in steps S103 and S105) and the graph creating steps (in steps S107 to S110) are completed, the result of the measurement is displayed on the display 10, and the report on the result of the measurement is outputted from the printer 9 (in step S111).

As described above, the display 10 displays the result of the measurement, as well as the caution or warning based on the results of the two tests on the result of the measurement.

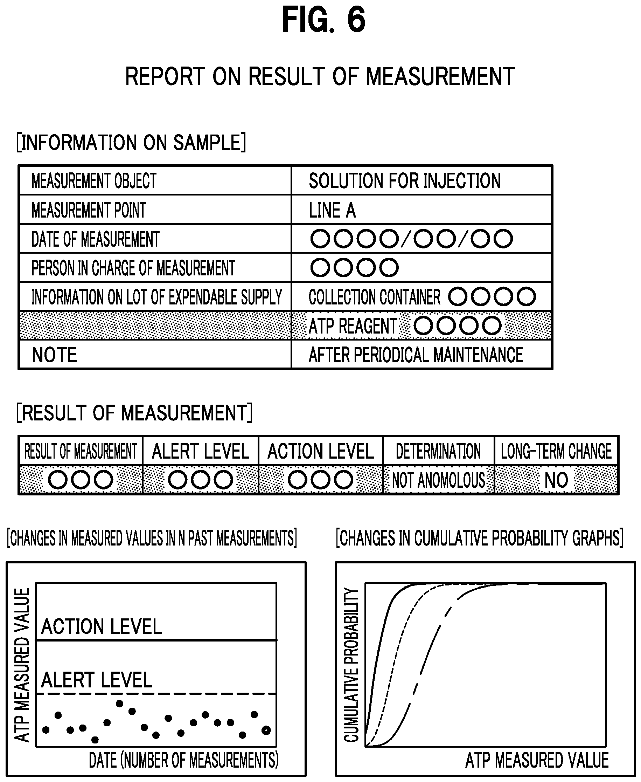

Meanwhile, it is desirable that in addition to the result of the measurement, a series of information needed to examine the result be printed on the report on the result outputted from the printer 9. FIG. 6 is a schematic diagram for explaining the report on the result in print of the microorganism measuring system 1 of the embodiment of the present invention.

As shown in FIG. 6, what are printed on the report on the result in print include: the information on the sample 2 (the sample information) inputted in step S100 in FIG. 4; the result of the measurement, inclusive of the set alert reference value (for the alert level) and action reference value (for the action level); the graph representing the changes in the results of the n past measurements (changes in the measured values in n past measurement) created in step S107 in FIG. 4; and the long-term change analysis graph (changes in the cumulative probability graphs) created in step S109 or step S110 in FIG. 4.

This makes it possible for the administrator to check the type of the measurement and the measurement conditions, to check whether or not the result of measuring the measurement object deviates from the reference values, as well as to examine whether or not the result of measuring the measurement object has changed over a long time. In addition, since the report on the result of the measurement in print is outputted from the printer 9 of the microorganism measuring system 1, the report on the result can be kept as in a printed form without a risk of the data being tampered, and the like.

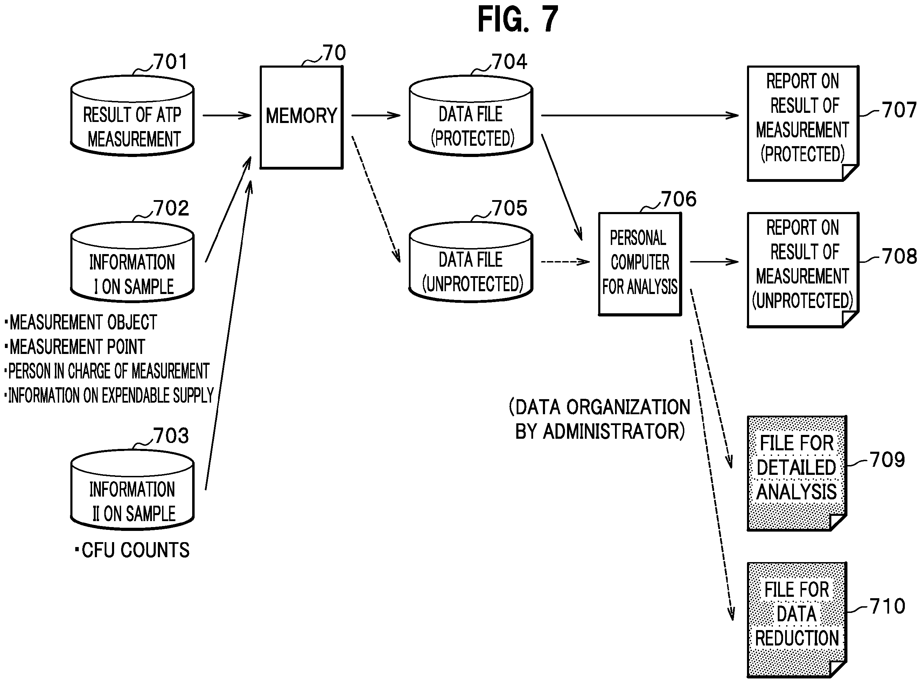

On the other hand, the report on the result can be kept as in an electronic-data format. Furthermore, the report on the result is prepared in a format which allows graph creation and the like in order for the administrator to examine the result of the measurement in various ways. FIG. 7 is a schematic diagram showing how data are stored in the microorganism measuring system 1 of the embodiment of the present invention, and how the output result files are configured.

The data stored in the memory 70 of the microorganism measuring system 1 are roughly classified into three categories. A first category is the data (reference sign 701) on the ATP measurement results obtained using the microorganism measuring system 1 (in step S2 in FIG. 3A or step S102 in FIG. 4). A second category is the information (reference sign 702) on the sample 2 which is inputted using the data input unit 8 each time the ATP measurement is performed (in step S3 in FIG. 3A or step S100 in FIG. 4). A third category is the information (reference sign 703) on the results of the measurements repeated for the CFU counting for setting the control reference values (in step S1 in FIG. 3A).

The data in the second category and the data in the third category are stored into the memory 70 by: being associated with the corresponding results of the ATP measurements; and being classified according to the types of measurement objects based on the information on the sample 2 inputted using the data input unit 8.

Once the ATP measurements are completed, the arithmetic unit 7 creates data files for the report on the result of the measurement by retrieving necessary data from the memory 70. The data files are identical. After that, one data file is saved in a protected format which makes data unable to be tampered (reference sign 704). The other data file is saved in an unprotected format which enables analyses, inclusive of graph creation, to be performed using the data file.

The report on the result of the measurement (original) in print is obtained by outputting the thus-created data file from the printer 9. Meanwhile, the two data files can be loaded into a personal computer for analysis (reference sign 706). The data file in the protected format can be saved or outputted (copied) depending on the necessity (reference sign 707).

On the other hand, the data file in the unprotected format can be used by the administrator for the purpose of data analyses and data reductions, because the use of the data file in the unprotected format enables analyses, inclusive of graph creation, to be performed using spreadsheet operation software, and to be outputted (copied) depending on the necessity (reference signs 708, 709, 710).

Although the foregoing descriptions have been provided for the embodiment of the present invention, the present invention is not limited to the embodiment, and can be carried out in various modes.

For example, in the process in FIG. 4, steps S103 to S106 and steps S107 to S110 may be performed in a reverse order or in parallel.

Moreover, although the ATP method is considered as the rapid microbiological method of the embodiment, the rapid microbiological method is not limited to the ATP method. The rapid microbiological method of the embodiment is applicable to all the microorganism detecting methods including: a direct counting method in which nucleic acids are stained, and the number of cells of microorganisms is counted under a microscope; and a method in which microorganisms are detected using autofluorescent substances as an indicator because the autofluorescent substances are in biological materials included in the microorganisms.

**Reference Signs List**

| | |
|---|---|
| 1 | microorganism measuring system |
| 2 | sample |
| 3 | filter collection unit |
| 4 | reagent dispenser |
| 5 | luminescence measurement unit |
| 6 | control unit |
| 7 | arithmetic unit |

| 8 | data input unit |
| 9 | printer |
| 10 | display |
| 30 | collection container |
| 30a | filter |
| 31 | collection container holder |
| 32 | filtration device |
| 32a | suction head |
| 32b | nut |
| 32c | ball screw shaft |
| 32d | motor |
| 32e | suction pump |
| 40 | nozzle |
| 41a | X,Y-axis actuator |
| 41b | Z-axis actuator |
| 42 | pump |
| 43 | reagent tube |
| 44 | reagent tube holder |
| 50 | measurement tube |
| 51 | measurement tube holder |
| 52 | photon counting unit |
| 60 | reagent dispensation controller |
| 61 | drive controller |
| 62 | filtration controller |
| 70 | memory |
| 100 | process stage |
| 110a | reaction liquid |
| 110b | blank sample liquid |
| 110c | ATP standard reagent |
| 110d | ATP luminescence reagent |

**Claims**

1. A microorganism measuring system comprising:

luminous reaction means for making ATP in cells of microorganisms in a sample cause luminous reaction by supplying a predetermined reagent to the microorganisms;
optical measurement means for measuring luminescence intensities of the microorganisms causing the luminous reaction;
arithmetic control means for converting measurement values of the luminescence intensities measured using the optical measurement means into ATP contents, storing the conversion results, and performing a statistical process, wherein
the arithmetic control means executes the steps of:

inputting results of CFU countings repeatedly performed using a surface plate culture method in a normal condition;
inputting results of ATP measurements repeatedly performed using the luminous reaction means and the optical measurement means at the same time as the CFU countings are repeatedly performed;
based on the results of the repeated CFU countings, determining a coefficient of a probability density function of CFU counts in the sample;
based on the results of the repeated ATP measurements, determining coefficients of a probability density function of ATP contents per CFU;
using the determined coefficients of the two probability density functions, determining a probability density function of ATP measured values in the normal condition;
performing a statistical test on appropriateness of the determined probability density function;
determining a probability density function of ATP measured values in a contaminated condition corresponding to CFU counts in the contaminated condition set in advance;

using the determined probability density function of the ATP measured values in the normal condition, calculating a alert reference value at which a probability of false positives is equal to or less than a probability set in advance;

using the determined probability density function of the ATP measured values in the contaminated condition, calculating an action reference value at which a probability of false negatives is equal to or less than a probability set in advance; and

each time the ATP measurement is performed, testing whether or not the ATP measured value satisfies any of the alert reference value and the action reference value.

2. The microorganism measuring system according to claim 1, wherein
a plurality of types of the probability density function of the ATP measured values are prepared, and
the arithmetic control means further executes the step of, based on the results of the repeated CFU countings, selecting one from the plurality of types of the probability density function of the ATP measured values.

3. The microorganism measuring system according to claim 1 or 2, wherein
the arithmetic control means determines the probability density function of the ATP measured values in the contaminated condition using the same coefficients as those in the probability density function of the ATP measured values in the normal condition.

4. The microorganism measuring system according to claim 1 or 2, wherein
the arithmetic control means determines the probability density function of the ATP measured values in the contaminated condition using a mean ATP content and a standard deviation, obtained in advance using standard strain of bacteria, as the coefficients.

5. The microorganism measuring system according to claim 1, wherein
the arithmetic control means further executes the step of testing whether or not a volume of the stored results of the repeated CFU countings and a volume of the stored results of the repeated ATP measurements are enough for the statistical test.

6. The microorganism measuring system according to claim 1, wherein
the arithmetic control means further uses a probability density function of ATP measured values calculated using the results of the measurements in a predetermined period, as the probability density function of the ATP measured values.

7. The microorganism measuring system according to claim 1,
further comprising a data input unit for inputting data and control conditions by manipulating keys,
wherein the arithmetic control means further uses the CFU counts in the contaminated condition, the probability of false positives, and the probability of false negatives, which are inputted using the data input unit.

8. The microorganism measuring system according to claim 1,
further comprising a data input unit for inputting data and control conditions by manipulating keys,
wherein when performing the ATP measurement, the arithmetic control means further uses information on the sample inputted using the data input unit.

9. The microorganism measuring system according to claim 1, wherein
the arithmetic control means further executes the step of performing an alert procedure or an action procedure based on a result of the step of testing whether or not the ATP measured value satisfies any of the alert reference value and the action reference value each time the ATP measurement is performed.

10. The microorganism measuring system according to claim 1,
further comprising printing means for outputting the results of the measurements in print,
wherein the arithmetic control means further executes the step of outputting the results of the ATP measurements and information needed to analyze the results of the ATP measurements using the printing means.

11. The microorganism measuring system according to claim 1, wherein
the arithmetic control means further executes the step of creating information needed to analyze the results of the ATP measurements, both in a protected format which disables the information from being tampered and in an unprotected format which enables the information to be analyzed.

12. A microorganism measuring method for a microorganism measuring system,

the microorganism measuring system comprising: luminous reaction means for making ATP in cells of microorganisms in a sample cause luminous reaction by supplying a predetermined reagent to the microorganisms; optical measurement means for measuring luminescence intensities of the microorganisms causing the luminous reaction; arithmetic control means for converting measurement values of the luminescence intensities measured using the optical measurement means into ATP contents, storing the conversion results, and performing a statistical process, wherein

the arithmetic control means executes the steps of:

inputting results of CFU countings repeatedly performed using a surface plate culture method in a normal condition;

inputting results of ATP measurements repeatedly performed using the luminous reaction means and the optical measurement means at the same time as the CFU countings are repeatedly performed;

based on the results of the repeated CFU countings, determining a coefficient of a probability density function of CFU counts in the sample;

based on the results of the repeated ATP measurements, determining coefficients of a probability density function of ATP contents per CFU;

using the determined coefficients of the two probability density functions, determining a probability density function of ATP measured values in the normal condition;

performing a statistical test on appropriateness of the determined probability density function;

determining a probability density function of ATP measured values in a contaminated condition corresponding to CFU counts in the contaminated condition set in advance;

using the determined probability density function of the ATP measured values in the normal condition, calculating a alert reference value at which a probability of false positives is equal to or less than a probability set in advance;

using the determined probability density function of the ATP measured values in the contaminated condition, calculating an action reference value at which a probability of false negatives is equal to or less than a probability set in advance; and

each time the ATP measurement is performed, testing whether or not the ATP measured value satisfies any of the alert reference value and the action reference value.

FIG. 1

# FIG. 2

REGION OF "NOT EXPECTED ANOMALOUS CONDITION"

REGION OF "NOT NORMAL CONDITION"

CUMULATIVE DISTRIBUTION OF NORMAL CONDITIONS
$F_S(x: \lambda, \mu, \sigma)$

CUMULATIVE DISTRIBUTION OF EXPECTED ANOMALOUS CONDITIONS
$F_S(x: \lambda_e, \mu_e, \sigma_e)$

AMOUNT OF ATP (amol)

CUMULATIVE PROBABILITY

1.0

0.0

$\varepsilon_1$

$\varepsilon_2$

$x_{c1}$

$x_{c2}$

△
CAUTION
REFERENCE VALUE

▲
WARNING
REFERENCE VALUE

## FIG. 3A

START

REPEAT CFU COUNTINGS IN NORMAL CONDITION — S1

REPEAT ATP MEASUREMENTS IN NORMAL CONDITION — S2

STORE RESULTS OF REPEATED CFU COUNTINGS AND RESULTS OF REPEATED ATP MEASUREMENTS — S3

CHECK VOLUME OF DATA ON RESULTS OF REPEATED CFU COUNTINGS AND VOLUME OF DATA ON RESULTS OF REPEATED ATP MEASUREMENTS "ENOUGH FOR G-TEST?" — S4

No

INCREASE NUMBER OF REPETITIONS OF CFU COUNTING AND NUMBER OF REPETITIONS OF ATP MEASUREMENT, IF DATA IS NOT ENOUGH — S5

Yes

SELECT ONE FROM PROBABILITY DISTRIBUTION MODELS OF ATP MEASURED VALUES BASED ON RESULTS OF REPEATED CFU COUNTINGS — S6

DETERMINE COEFFICIENT OF PROBABILITY DENSITY FUNCTION OF CFU COUNTS INCLUDED IN SAMPLE — S7

DETERMINES COEFFICIENTS OF PROBABILITY DENSITY FUNCTION OF AMOUNTS OF ATP INCLUDED IN EACH CFU — S8

DETERMINE PROBABILITY DENSITY FUNCTION OF ATP MEASURED VALUES IN NORMAL CONDITION — S9

SUBJECT PROBABILITY MODEL OF ATP MEASURED VALUES IN NORMAL CONDITION TO G-TEST — S10

No

SELECT ANOTHER PROBABILITY MODEL IF RESULT OF G-TEST IS FAILURE — S11

Yes

A

# FIG. 3B

Ⓐ

STORE EXPECTED VALUE FOR CFU COUNT IN CONTAMINATED CONDITION, AS WELL AS PROBABILITY OF FALSE POSITIVES AND PROBABILITY OF FALSE NEGATIVES TOLERATED IN MICROORGANISM CONTROL ──S12

DETERMINE PROBABILITY DENSITY FUNCTION OF ATP MEASURED VALUES IN CONTAMINATED CONDITION ──S13

──S14
DETERMINE CAUTION REFERENCE VALUE "REFERENCE VALUE EQUAL TO OR LESS THAN TOLERATED PROBABILITY OF FALSE POSITIVES CAN BE SET?"

No → ──S15 DETERMINE CAUTION REFERENCE VALUE BY TESTING MULTIPLE TIMES, IF CONDITION IS NOT SATISFIED

Yes

──S16
DETERMINE WARNING REFERENCE VALUE "REFERENCE VALUE EQUAL TO OR LESS THAN TOLERATED PROBABILITY OF FALSE NEGATIVES CAN BE SET?"

No → ──S17 DETERMINE WARNING REFERENCE VALUE BY TESTING MULTIPLE TIMES, IF CONDITION IS NOT SATISFIED

Yes

STORE CAUTION REFERENCE VALUE AND WARNING REFERENCE VALUE ──S18

END

# FIG. 4

```
( START )
    │
    ▼
┌────────────────────────────┐
│ STORE INFORMATION ON SAMPLE │──S100
└────────────────────────────┘
    │
    ▼
┌────────────────────────────┐
│        MEASURE ATP          │──S101
└────────────────────────────┘
    │
    ▼
┌────────────────────────────┐
│   STORE RESULT OF MEASUREMENT │──S102
└────────────────────────────┘
    │
    ▼
```

S103
SUBJECT RESULT OF MEASUREMENT TO TEST 1 "RESULT OF ATP MEASUREMENT < WARNING REFERENCE VALUE"

No → S104 ACTIVATE WARNING

Yes
    ▼

S105
SUBJECT RESULT OF MEASUREMENT TO TEST 2 "RESULT OF ATP MEASUREMENT < CAUTION REFERENCE VALUE"

No → S106 DISPLAY CAUTION

Yes
    ▼

S107
CREATE GRAPH REPRESENTING CHANGES IN ATP MEASURED VALUES OF N PAST MEASUREMENTS AND GRAPH REPRESENTING CUMULATIVE PROBABILITY
    │
    ▼

S108
CHECK NUMBER OF DATA "CURRENT CUMULATIVE NUMBER OF MEASUREMENTS = X MULTIPLIED BY n (X=INTEGER)"

No → S110 CREATE LONG-TERM CHANGE ANALYSIS GRAPH (FORMAT B)

Yes
    ▼

S109
CREATE AND STORE LONG-TERM CHANGE ANALYSIS GRAPH (FORMAT A)
    │
    ▼

S111
OUTPUT RESULT
    │
    ▼
( END )

27

# FIG. 5A

## FORMAT A

NUMBER OF MEASUREMENTS
DATA ON MEASUREMENTS
DIVISION OF DATA FOR STORAGE

EP 3 121 263 A1

# FIG. 5B

## FORMAT B

DIVISION OF DATA FOR STORAGE
NUMBER OF MEASUREMENTS
DATA ON MEASUREMENTS
DIVISION OF DATA FOR OUTPUT

$1 \cdots n \cdots 2n \cdots 3n \cdots 3n+k(k<n)$

CUMULATIVE PROBABILITY

AMOUNT OF ATP (amol)

L1
L2
L3
L4

# FIG. 6

## REPORT ON RESULT OF MEASUREMENT

[INFORMATION ON SAMPLE]

| MEASUREMENT OBJECT | SOLUTION FOR INJECTION |
|---|---|
| MEASUREMENT POINT | LINE A |
| DATE OF MEASUREMENT | ○○○○/○○/○○ |
| PERSON IN CHARGE OF MEASUREMENT | ○○○○ |
| INFORMATION ON LOT OF EXPENDABLE SUPPLY | COLLECTION CONTAINER ○○○○ |
| | ATP REAGENT ○○○○ |
| NOTE | AFTER PERIODICAL MAINTENANCE |

[RESULT OF MEASUREMENT]

| RESULT OF MEASUREMENT | ALERT LEVEL | ACTION LEVEL | DETERMINATION | LONG-TERM CHANGE |
|---|---|---|---|---|
| ○○○ | ○○○ | ○○○ | NOT ANOMOLOUS | NO |

[CHANGES IN MEASURED VALUES IN N PAST MEASUREMENTS]

[CHANGES IN CUMULATIVE PROBABILITY GRAPHS]

# FIG. 7

- RESULT OF ATP MEASUREMENT — 701
- INFORMATION I ON SAMPLE — 702
  - ·MEASUREMENT OBJECT
  - ·MEASUREMENT POINT
  - ·PERSON IN CHARGE OF MEASUREMENT
  - ·INFORMATION ON EXPENDABLE SUPPLY
- INFORMATION II ON SAMPLE — 703
  - ·CFU COUNTS
- MEMORY — 70
- DATA FILE (PROTECTED) — 704
- DATA FILE (UNPROTECTED) — 705
- PERSONAL COMPUTER FOR ANALYSIS — 706
- REPORT ON RESULT OF MEASUREMENT (PROTECTED) — 707
- REPORT ON RESULT OF MEASUREMENT (UNPROTECTED) — 708
- (DATA ORGANIZATION BY ADMINISTRATOR)
- FILE FOR DETAILED ANALYSIS — 709
- FILE FOR DATA REDUCTION — 710

EP 3 121 263 A1

## INTERNATIONAL SEARCH REPORT

| International application No. |
| --- |
| PCT/JP2015/057544 |

| A.   CLASSIFICATION OF SUBJECT MATTER |
| --- |
| *C12M1/34*(2006.01)i, *C12Q1/06*(2006.01)i, *G01N21/76*(2006.01)i, *G01N21/77*(2006.01)i, *G01N33/50*(2006.01)i |
| According to International Patent Classification (IPC) or to both national classification and IPC |

| B.   FIELDS SEARCHED |
| --- |
| Minimum documentation searched (classification system followed by classification symbols)<br>C12M1/34, C12Q1/06, G01N21/76, G01N21/77, G01N33/50 |
| Documentation searched other than minimum documentation to the extent that such documents are included in the fields searched |
| Electronic data base consulted during the international search (name of data base and, where practicable, search terms used)<br>JSTPlus/JMEDPlus/JST7580(JDreamIII), CAplus/MEDLINE/BIOSIS/WPIDS(STN) |

| C.   DOCUMENTS CONSIDERED TO BE RELEVANT | | |
| --- | --- | --- |
| Category* | Citation of document, with indication, where appropriate, of the relevant passages | Relevant to claim No. |
| A | JP 2009-17852 A  (Hitachi Plant Technologies, Ltd.),<br>29 January 2009 (29.01.2009),<br>claims; paragraph [0039]<br>(Family: none) | 1-12 |
| A | JP 2012-211785 A  (Hitachi Plant Technologies, Ltd.),<br>01 November 2012 (01.11.2012),<br>claims; paragraph [0081]; fig. 6<br>& US 2012/0250024 A1     & EP 2505990 A1 | 1-12 |
| A | JP 9-182600 A  (Kikkoman Corp.),<br>15 July 1997 (15.07.1997),<br>example 9; paragraph [0097]; fig. 11<br>& US 5891702 A           & EP 781851 A2 | 1-12 |

☒   Further documents are listed in the continuation of Box C.      ☐   See patent family annex.

| * | Special categories of cited documents: |
| --- | --- |
| "A" | document defining the general state of the art which is not considered   to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search<br>    02 June 2015 (02.06.15) | Date of mailing of the international search report<br>    16 June 2015 (16.06.15) |
| --- | --- |
| Name and mailing address of the ISA/<br>    Japan Patent Office<br>    3-4-3,Kasumigaseki,Chiyoda-ku,<br>    Tokyo 100-8915,Japan | Authorized officer<br><br>Telephone No. |

Form PCT/ISA/210 (second sheet) (July 2009)

**INTERNATIONAL SEARCH REPORT**

International application No.

PCT/JP2015/057544

C (Continuation).    DOCUMENTS CONSIDERED TO BE RELEVANT

| Category* | Citation of document, with indication, where appropriate, of the relevant passages | Relevant to claim No. |
|---|---|---|
| A | SUTHERLAND, A. D. et al., The Biotrace method for estimating bacterial numbers in milk by bioluminescence, Journal of the Society of Dairy Technology, 1994, Vol.47, No.4, pp.117-120 | 1-12 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3773151 B
- JP 2006081506 A

- JP 5030015 B